# EUROPEAN PATENT SPECIFICATION

(11) **EP 4 149 608 B1**
(45) Date of publication and mention of the grant of the patent: **01.05.2024**
(21) Application number: 21725502.5
(22) Date of filing: 12.05.2021
(51) Int. Cl.: A61N 1/05, A61N 1/372, G16H 50/50

(54) **SPINAL CORD STIMULATION SYSTEM**
RÜCKENMARKSSTIMULATIONSSYSTEM
SYSTÈME DE STIMULATION DE MOELLE ÉPINIÈRE

(30) Priority: 12.05.2020 EP 20382387
(43) Date of publication of application: 22.03.2023
(73) Proprietor: Surgicen SLU, 46230 Alginet, Valencia (ES); García Vitoria, Carles, 46003 Valencia (ES)
(72) Inventor: DURÁ CANTERO, José Luis, 46014 Valencia (ES); GARCÍA VITORIA, Carles, 46003 Valencia (ES)
(74) Representative: Hoffmann Eitle
(86) International application number: PCT/EP2021/062575
(87) International publication number: WO 2021/228916

(56) References cited:
- WO-A1-2014/135592
- WO-A1-2020/186153
- US-A1- 2006 224 219
- US-A1- 2017 095 667

## Description

### FIELD OF THE INVENTION

The present invention refers to the medical field. Specifically, the present invention refers to a spinal cord stimulation system for spinal cord stimulation (SCS) which comprises: a) An electrode (1), consisting essentially of a plurality of poles (2), suitable for being inserted inside the intradural space (3) through the dura mater (4) preferably via needle-puncture (5) of the skin; b) unit (6), connected to the electrode, suitable for generating electric current and reading the impedance; and c) a computer comprising a processing unit (10).

### STATE OF THE ART

SCS is an effective therapy for the treatment of chronic and intractable pain including diabetic neuropathy, failed back surgery syndrome, complex regional pain syndrome, phantom limb pain, ischemic limb pain, refractory unilateral limb pain syndrome, postherpetic neuralgia and acute herpes zoster pain. Another pain condition that is a potential candidate for SCS treatment is Charcot-Marie-Tooth (CMT) disease, which is associated with moderate to severe chronic extremity pain. SCS therapy consists of the electrical stimulation of the spinal cord to 'mask' pain. The gate theory proposed in 1965 by Melzack and Wall provided a theoretical construct to attempt SCS as a clinical treatment for chronic pain. This theory postulates that activation of large diameter, myelinated primary afferent fibres suppresses the response of dorsal horn neurons to input from small, unmyelinated primary afferents. A simple SCS system consists of three different parts. First, electrodes are implanted to deliver stimulation pulses to the tissue (the pulses are delivered to the tissue through poles located on those electrodes). Second, an electrical pulse generator implanted while is connected to the electrodes via wires, and third a remote control to adjust the stimulus parameters such as pulse width and pulse rate. U.S. Pat. 10/335,596 discloses systems, devices, and methods for neurostimulation using a combination of implantable and external devices to treat pain.

More than 50,000 stimulators are implanted annually in the world. Almost all of them are placed in the epidural space. The epidural space is the area between the dura mater and the vertebral wall, containing fat and small blood vessels. The epidural space is located just outside the dural sac which surrounds the spinal cord, nerve roots and is filled with cerebrospinal fluid.

The use of stimulators in the epidural space is associated with some problems, their low energy efficiency being the main one. This is because, if the electrode poles are located in the epidural space, a large part of the current generated by the pulse generator does not reach the spinal cord because of the intervening tissues, such as the dura mater and epidural fat, which would be placed between the poles and the spinal cord. The presence of electrode poles inside the intradural space could solve this problem, since the resistance to the passage of current is close to zero since the cerebrospinal fluid acts like a conductor.

On the other hand, there is a scientific consensus on the idea that it is desirable to minimize the invasiveness of the procedures to be performed, and the field of spinal stimulation is not an exception. In this regard, the surgical approach aimed at delivering current inside the epidural space involves the dissection of skin and tissues, their opening and the insertion of the devices directly in the desired location. Consequently, this approach should be substituted by other less invasive procedures such as a percutaneous approach wherein the electrodes are directed to the epidural space through a needle without requiring an incision or tissue dissection.

Some attempts have been made to conceptualize the possible placement of electrodes in the subdural space in order to maximize its energy performance. However, in all cases, this procedure involved performing a surgical technique: opening of skin and tissues, their dissection, sometimes the removal of bone sheets until reaching the dural sac and, once exposed, dissection of its layers until reaching the subarachnoid space to place the implant. Moreover, there is the risk of cerebrospinal fluid leakage through the surgical dural gap or hematoma. Consequently, it is recommended to avoid the surgical manipulation of a tissue such as dural tissue, which forms a layer about 300 micrometres thick.

So, in summary, there is an unmet medical need of finding more efficient and less invasive SCS techniques to treat chronic and intractable pain. To date, there are no electrodes designed to be inserted percutaneously into the subarachnoid or intradural space. Current electrodes are designed for the stimulation of the spinal cord by means of stimulators placed in the epidural space which give rise to the drawbacks explained above.

The present invention aims to provide a solution to this problem and it is herein provided an innovative and minimally invasive medical device for spinal cord stimulation which provides a high energy efficiency. The invention is defined in the appended claims. Aspects, embodiments and examples disclosed herein which do not fall within the scope of the appended claims do not form part of the invention and are merely provided for illustrative purposes. Furthermore, the methods presented in the present description are not claimed as such but are useful for understanding of the claimed subject matter, in particular since the methods can be performed by the claimed subject-matter.

### DESCRIPTION OF THE INVENTION

The present invention refers to a spinal cord stimulation system for spinal cord stimulation (SCS) which comprises:
a) An electrode (1), consisting essentially of a plurality of poles (2), suitable for being inserted inside the intradural space (3) through the dura mater (4) preferably via needle-puncture (5) of the skin;
b) Unit (6), connected to the electrode, suitable for generating electric current and reading the impedance; and
c) A computer comprising a processing unit (10).

Thus, the spinal cord stimulation system of the invention comprises an electrode (1) with a essential technical feature because its reduced diameter makes it suitable for being inserted inside the intradural space (3) through the dura mater (4), preferably via needle-puncture (5) of the skin (percutaneous technique).

The spinal cord stimulation system of the invention is associated with some technical advantages:
- It provides a high energy efficiency: Since the electrode is directly placed inside the intradural space, a large amount of the current generated by the pulse generator reach the spinal cord because there are not structures, like the dura mater, interrupting the passage of current. In fact, in the intradural space, the resistance to the passage of current is close to zero since the cerebrospinal fluid acts like a current conductor. Thus, the implantation of the stimulator inside the intradural space (3), rather than in the epidural space, directly impacts on the energy efficiency of the system. The intradural space (3) is the area inside the dura mater (4) (inside the dural sac) which is filled with cerebrospinal fluid (CSF) surrounding the medulla. Thus, the stimulation inside the intradural space (3) would be more energy efficient since it could be possible to take advantage of the conductivity of the CSF to exert its action, reducing energy consumption and allowing greater tissue penetration. Moreover, electrical current does not need to go through the dura mater (4) to reach the spinal cord, since the electrical current is directly applied in the intradural space (3). By means of the stimulation in the intradural space, the expected energy consumption is hundreds of times lower (as compared with the stimulation in the epidural space) due to the presence of CSF and no other structures that disperse energy.
- Said high energy efficiency enables the physician to use lower current intensities without jeopardizing the efficiency of the treatment. The use of said low intensities gives rise to the possibility of designing an electrode (1) with a reduced diameter.
- At the same time, said reduced diameter of the electrode (1) gives rise to the possibility of designing a minimally invasive medical devise because the electrode can be inserted in the patient preferably via needle-puncture (5) of the skin. This technique is a relatively minimally invasive procedure involving superficial and controlled puncturing of the skin by rolling with miniature fine needles (5), thus avoiding the need of dissecting skin, fat, muscles and minimal amounts of bone from the spine.
- Finally, it is highly important to consider that the spinal cord stimulation system of the invention has been specifically configured to place a "sentinel" pole inside the intradural space (3) but fixed to or in contact with the inner part of the dura mater-arachnoid (8) wherein the current is to be applied. So, in the context of the present invention, it is of utmost importance to consider that the pole used to apply the electric current is not freely loose within the intradural space but the electric current is applied once the pole is fixed to the inner part of the dura mater-arachnoid (8), with the objective to avoid that the patient perceives unusual or weird stimulations and also to be able to apply a constant electric current to the specific target anatomical area.

So, the first embodiment of the present invention refers to a spinal cord stimulation system (spinal cord stimulation system of the invention) characterized by comprising:
a. An electrode (1) with a diameter ≤ 1.3 mm, consisting essentially of a plurality of poles (2), suitable for being inserted inside the intradural space (3);
b. Unit (6), connected to the electrode, configured to systematically read the impedance of the poles (2) when the electrode (1) is going through the dura matter (4), and to generate electric current; and
c. A computer comprising a processing unit (10) configured to:
   i. Receive, from the unit (6) continuous impedance readings of each pole when the electrode (1) is going through the dura matter (4),
   ii. Process the impedance readings received for finding a statistically significant variation or deviation of the impedance, and
   iii. Provide an output through a terminal when a statistically significant variation or deviation of the impedance is identified, wherein a statistically significant reduction of the impedance of a specific pole (2) indicates that the pole has gone through the dura mater (4) and is placed in the intradural space at the inner part of the dura mater-arachnoid (8), wherein the electrical current is to be applied.

The identification of a statistically significant reduction of the impedance indicates that a specific pole ("sentinel" pole) has gone through the dura mater (4) and is placed in the intradural space at the inner part of the dura mater-arachnoid (8), wherein the electrical current is to be applied.

So, according to the first embodiment of the present invention, the spinal cord stimulation system of the invention comprises three main elements: a) electrode (1), b) unit (6) and c) computer comprising a processing unit (10).

When the electrode (1) is being placed inside the intradural space, unit (6) is activated with the objective of reading the impedance. The impedance values indirectly indicate to the clinician the position of the poles (2) when they are being inserted because a statistically significant variation of the impedance is expected when each pole goes through the dura mater (4) and gets into/out the intradural space. So, unit (6) continuously reads the impedance and the impedance readings are received by the processing unit (10). The processing unit (10) process the impedance readings just received for finding a statistically significant variation or deviation of the impedance (when the poles move from the outer to the inner part of the intradural space or vice versa) and provide an output through a terminal when a statistically significant reduction of the impedance is identified.

In a preferred embodiment the terminal is a display device for providing an output regarding the statistically significant variation or deviation of the impedance which has been identified. However, in the context of the present invention, the term terminal comprises any tool designed for providing an output regarding the substantial variation or deviation of the impedance which has been identified, like sound, noise, light, etc.

When the poles move from the outer to the inner part of the intradural space, or vice versa, a statistically significant variation or deviation of the impedance is clearly noticed since the impedance inside and outside the intradural space is very different. In fact, for instance, the electrical conductivity (which is the inverse of the resistivity) of the cerebrospinal fluid is around 1,700 S/m and the electrical conductivity of the dura mater is around 0,030 S/m. So, when the poles move from the outer to the inner part of the intradural space, a substantial reduction of the impedance is observed due the very low resistance of the cerebrospinal fluid which is inside the intradural space. Conversely, when the poles move from the inner to the outer part of the intradural space, a substantial increase of the impedance is observed due the higher resistance of the tissues which are out of the intradural space like the dura mater, fat, bone, etc. In general terms, a resistance lower than 50 Ohms indicates that the poles are already inside the intradural space.

As explained above, the variation or deviation of the impedance indicates that a specific pole has just gone through the dura matter (4), has reached the proximal part of the intradural space, and is fixed to the inner part of the dura mater-arachnoid (8), wherein the current is to be applied. So, the variation or deviation of the impedance when the electrode is being inserted, is an indication for the clinician that the pole is just placed at the desired position (inner part of the dura mater-arachnoid (8)), to apply the electrical current.

In a preferred embodiment, the spinal cord stimulation system of the invention further comprises an external fixation to the tissue (9) to fasten the electrode (1) once the pole is placed at the desired position, fixed to the inner part of the dura mater-arachnoid (8), wherein the electrical current is to be applied. This way, the clinician could apply the treatment with the guarantee that the pole to be stimulated will not move from the desired position.

In a preferred embodiment, the electrode has a diameter ≤ 1.3 mm. In a preferred embodiment, the electrode has a diameter from 0.15mm to 0.9mm. This is the diameter required by the electrode to be inserted inside the intradural space (3) through the dura mater (4) preferably via needle-puncture of the skin (5). Thus, it has a diameter configured to pass through a relatively low-calibre needle (5) to simplify the intrathecal approach, minimize the size of the dural perforation, and avoid CSF leaks. Assuming low-frequency stimulation will be used, the electrode could have a diameter of 0.15 mm, which would allow the use a very fine lead wire (and easier to implant), preferably covered by an insulating layer.

In a preferred embodiment, the diameter of the needle is ≤ 1.31mm, preferably ≤ 0.91 mm.

In a preferred embodiment, the electrode (1) comprises means, for instance a coating or cover (7), that facilitates macrophage adhesion and fibrous reaction, to be fixed to the ligamentous or muscular structures of the extradural space once at least two poles have reached the intradural space. So, it comprises at least two stimulation poles to create the intrathecal electric field. According to this preferred embodiment, once the poles have reached the intradural space (this is indicated by the impedance shown by the unit (6)), the electrode can be fixed to the ligamentous or muscular structures of the extradural space to avoid the backward movement of the electrode and thereby prevent the poles from falling out of the intradural space. Since an intrathecal subthreshold stimulation would be used, a due to the energy efficiency of the system, it is possible to propose the mere presence of 2 stimulation poles. Currently, the epidural electrodes have 4, 8 or 16 poles separated by a few millimetres from each other due to the need to "fight" against the dispersion of energy creating complicated combinations of stimulation.

In a preferred embodiment, each pole can be programmed to be active or passive. This gives the possibility of selecting the area to be stimulated inside the subarachnoid or intradural space.

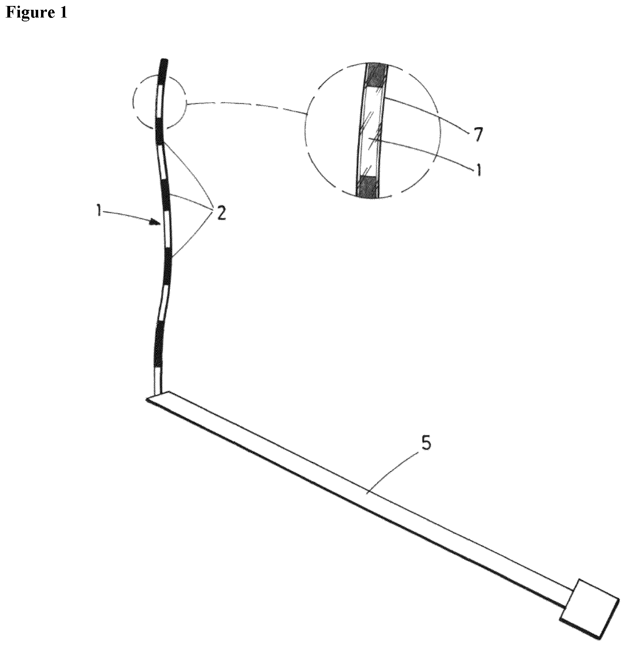

In a preferred embodiment, the electrode is flexible (see **Figure 1**) in order to easily move throughout the intradural space (3) to the desired metameric level for stimulation and avoid damages in the area. Thus, it is flexible and with a minimum of firmness that helps to advance it to the desired metameric level for stimulation.

In a preferred embodiment the tip of the electrode is completely rounded.

In a preferred embodiment, the electrode is covered with a cover (7) that prevents trauma, for example silicone rubber elastomer.

An example of the present description refers to a computer implemented method for assessing whether an electrode pole (2) has gone through the dura mater (4) and it is placed in the intradural space at the inner part of the dura mater-arachnoid (8) which comprises: a) Systematically reading the impedance when the electrode (1) is going through the dura mater (4), and b) wherein the identification of a statistically significant reduction of the impedance of a specific pole (2) indicates that the pole has gone through the dura mater (4) and is placed in the intradural space at the inner part of the dura mater-arachnoid (8), wherein the electrical current is to be applied.

An example of the present description refers to a computer program comprising instructions to cause the stimulation system of the invention to execute the steps of the methods described above.

An example of the present description refers to a computer-readable medium having stored thereon the above cited computer program.

An example of the present description refers to a method for spinal cord stimulation or for treating pain in a patient which comprises using the spinal cord stimulation system of the invention, wherein a specific pole is placed in the intradural space at the inner part of the dura mater-arachnoid (8), wherein the electrical current is to be applied.

The spinal cord stimulation system of the invention could have two more exemplary alternative configurations:
It may comprise:
a. An electrode **(1)** with a diameter ≤ 1.3 mm, consisting essentially of a plurality of poles **(2),** suitable for being inserted inside the intradural space **(3);**
b. Unit **(6),** connected to the electrode, configured to systematically read the impedance of the poles **(2)** when the electrode **(1)** is going through the dura matter **(4),** and to generate electric current; and
c. An external fixation to the tissue **(9)** to fasten the electrode **(1)** once the pole is placed in the intradural space at the inner part of the dura mater-arachnoid **(8),** wherein the electrical current is to be applied.

Alternatively, it may comprise:
a. An electrode **(1)** with a diameter ≤ 1.3 mm, consisting essentially of a plurality of poles **(2),** suitable for being inserted inside the intradural space **(3);**
b. Unit **(6),** connected to the electrode, configured to systematically read the impedance of the poles **(2)** when the electrode **(1)** is going through the dura matter **(4),** and to generate electric current; and.
c. A computer comprising a processing unit **(10)** configured to:
   i. Receive, from the unit **(6)** continuous impedance readings of each pole when the electrode **(1)** is going through the dura matter **(4),**
   ii. Process the impedance readings received for finding a statistically significant variation or deviation of the impedance between the impedance readings, and
   iii. Give introductions to unit **(6)** to apply electric current when a statistically significant variation or deviation of the impedance is identified, wherein a statistically significant reduction of the impedance of a specific pole **(2)** indicates that the pole has gone through the dura mater **(4)** and is placed in the intradural space at the inner part of the dura mater-arachnoid **(8),** wherein the electrical current is to be applied.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.

The present invention is better illustrated in **Figures 1** to **4** wherein the following references are cited:

| **Figure reference** | **Technical feature** |
|---|---|
| (1) | electrode |
| (2) | plurality of poles |
| (3) | subarachnoid or intradural space |
| (4) | dura mater |
| (5) | needle |
| (6) | unit |
| (7) | electrode cover |
| (8) | pole placed in the intradural space at the inner part of the dura mater-arachnoid |
| (9) | electrode fixation to the tissue |
| (10) | computer comprising a processing unit |

### Brief description of the figures

**Figure 1****.** It shows the electrode of the invention (1), which comprises a plurality of poles (2), and a cover (7).

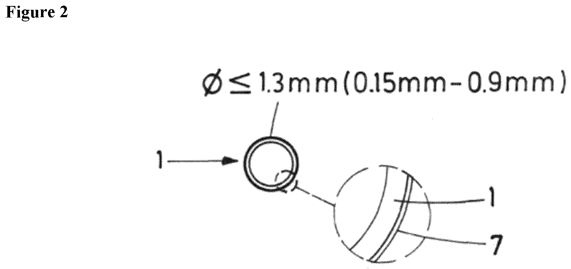

**Figure 2****.** Is shows a sagittal plane illustrating the diameter of the electrode which is ≤ 1.3 mm, preferably between 0.15mm to 0.9mm, in order to be inserted inside the intradural space. The electrode cover (7) is also represented in this figure.

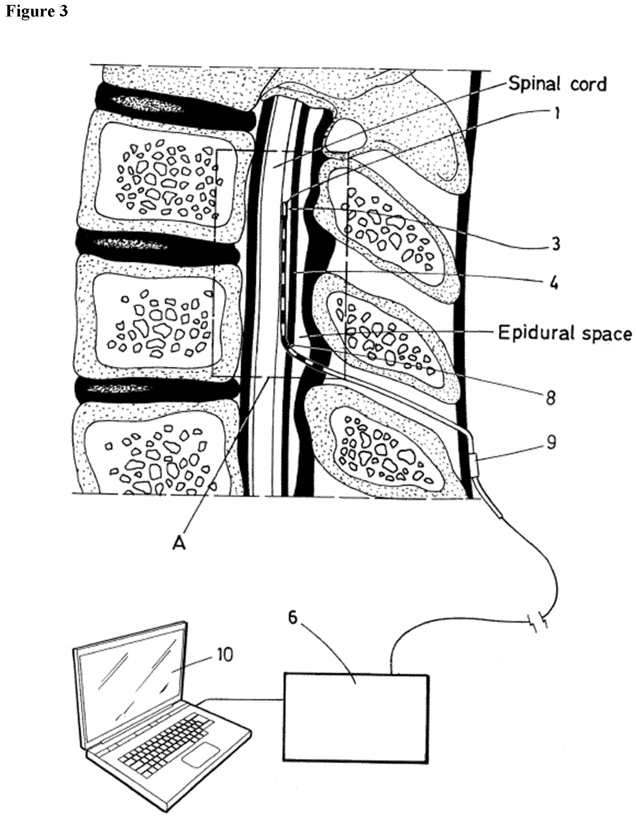

**Figure 3**. It shows the electrode of the invention (1) inserted in the intradural space (3), (and not just in the epidural space), through the dura mater (4), preferably by means of a needle (5) which has a dimeter ≤ 1.31mm. It is represented the position where the pole is placed in the intradural space at the inner part of the dura mater-arachnoid (8), wherein the electrical current is to be applied. The computer comprising a processing unit (10) is also represented.

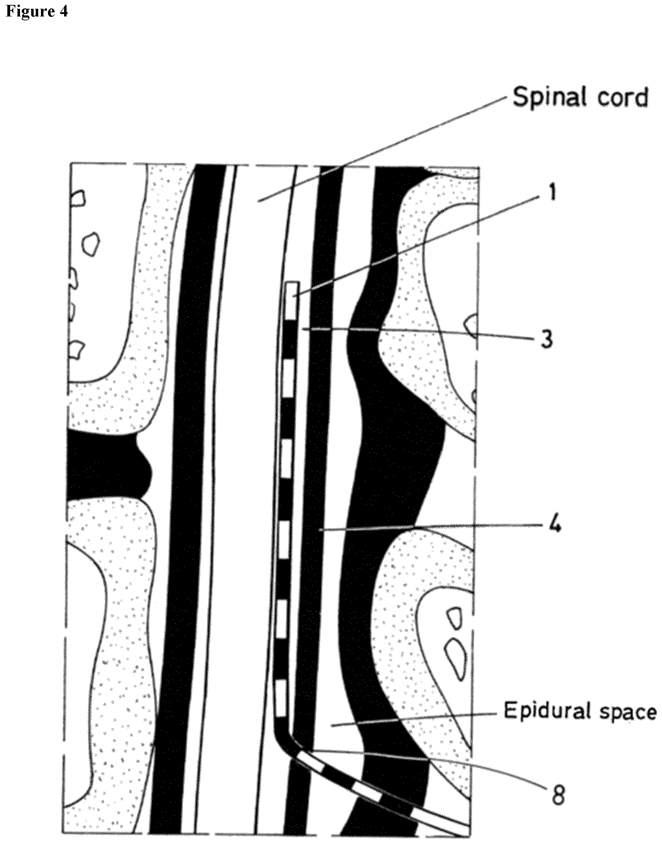

**Figure 4**. It shows a close view wherein a crucial aspect of the invention is illustrated. The electrode of the invention (1) inserted in the intradural space (3), (and not just in the epidural space), through the dura mater (4) and the pole is in the intradural space at the inner part of the dura mater-arachnoid (8), wherein the electrical current is to be applied.

## Claims

1. Spinal cord stimulation system which comprises:
a. An electrode (1) with a diameter ≤ 1.3 mm, consisting essentially of a plurality of poles (2), suitable for being inserted inside the intradural space (3);
b. Unit (6), connected to the electrode, configured to systematically read the impedance of the poles (2) when the electrode (1) is going through the dura matter (4), and to generate electric current; and
c. A computer comprising a processing unit (10) configured to:
i. Receive, from the unit (6), continuous impedance readings of each pole when the electrode (1) is going through the dura matter (4),
ii. Process the impedance readings received for finding a statistically significant variation or deviation of the impedance, and
iii. Wherein the computer comprising a processing unit (10) **is characterized in that it is further configured** to provide an output through a terminal and give instructions to unit (6) to apply electric current inside the intradural space (3), when a statistically significant reduction of the impedance of a specific pole (2) is identified, which indicates that the pole (2) has gone through the dura mater (4) and is placed in the intradural space (3) at the inner part of the dura mater-arachnoid (8).

2. Spinal cord stimulation system, according to claim 1, further comprising an external fixation to the tissue (9) to fasten the electrode (1) once the pole is placed in the intradural space at the inner part of the dura mater-arachnoid (8), wherein the electrical current is to be applied.

3. Spinal cord stimulation system, according to any of the previous claims, wherein the processing unit (10) comprises at least a computer program comprising instructions to receive, from the unit (6), continuous impedance readings of each pole, process the impedance readings received for finding a statistically significant variation or deviation of the impedance, and provide an output through a terminal, and/or give instructions to unit (6) to apply electric current, when a statistically significant reduction of the impedance is identified.

4. Spinal cord stimulation system, according to any of the previous claims, wherein the electrode (1) with a diameter ≤ 1.3 mm, consisting essentially of a plurality of poles (2), is configured to be inserted inside the intradural space (3) through the dura mater (4) via needle-puncture (5) of the skin.

5. Spinal cord stimulation system, according any of the previous claims, wherein the electrode (1) has a diameter from 0.15mm to 0.9mm.

6. Spinal cord stimulation system, according any of the previous claims, wherein the electrode (1) is flexible in order to easily move throughout the intradural space to the desired metameric level for stimulation and avoid damages in the area.

7. Spinal cord stimulation system, according any of the previous claims, wherein the electrode (1) is covered with a cover (7) that prevents trauma, for example silicone rubber elastomer.

8. Spinal cord stimulation system, according to any of the previous claims, wherein the diameter of the needle (5) is ≤ 1.31mm, preferably ≤ 0.91 mm.

9. Spinal cord stimulation system, according to any of the previous claims, wherein each pole can be programmed to be active or passive.

## Patentansprüche

1. Rückenmarksstimulationssystem, welches Folgendes umfasst:
a. eine Elektrode (1) mit einem Durchmesser ≤ 1,3 mm, welche im Wesentlichen aus einer Vielzahl von Polen (2) besteht, welche dafür geeignet sind, innerhalb des intraduralen Raums (3) eingeführt zu werden;
b. eine Einheit (6), welche mit der Elektrode verbunden ist, welche dazu ausgebildet ist, die Impedanz der Pole (2) systematisch zu lesen, wenn die Elektrode (1) durch die Dura mater (4) durchgeht, und einen elektrischen Strom zu erzeugen; und
c. einen Computer, welcher eine Verarbeitungseinheit (10) umfasst, welche dazu ausgebildet ist:
i. kontinuierliche Impedanzablesungen jedes Pols aus der Einheit (6) zu empfangen, wenn die Elektrode (1) durch die Dura mater (4) durchgeht,
ii. die empfangenen Impedanzablesungen zu verarbeiten, um eine statistisch signifikative Variation oder Abweichung der Impedanz zu finden, und
iii. wobei der Computer, welcher eine Verarbeitungseinheit (10) umfasst, **dadurch gekennzeichnet ist, dass** er zusätzlich dazu ausgebildet ist, eine Ausgabe über ein Endgerät bereitzustellen und der Einheit (6) Anweisungen zu geben, um einen elektrischen Strom innerhalb des intraduralen Raums (3) anzulegen, wenn eine statistisch signifikante Verringerung der Impedanz eines spezifischen Pols (2) identifiziert wird, was anzeigt, dass der Pol (2) durch die Dura mater (4) durchgegangen ist, und im intraduralen Raum im inneren Teil der Dura mater-Spinnwebhaut (8) platziert ist.

2. Rückenmarksstimulationssystem nach Anspruch 1, zusätzlich umfassend eine äußere Fixierung zum Gewebe (9), um die Elektrode (1) zu befestigen, sobald der Pol im intraduralen Raum im inneren Teil der Dura mater-Spinnwebhaut (8) platziert ist, worin der elektrische Strom anzulegen ist.

3. Rückenmarksstimulationssystem nach einem der vorhergehenden Ansprüche, wobei die Verarbeitungseinheit (10) mindestens ein Computerprogramm umfasst, welches Anweisungen umfasst, um kontinuierliche Impedanzablesungen jedes Pols aus der Einheit (6) zu empfangen, die empfangenen Impedanzablesungen zu verarbeiten, um eine statistisch signifikative Variation oder Abweichung der Impedanz zu finden, und eine Ausgabe über ein Endgerät bereitzustellen, und/oder der Einheit (6) Anweisungen zu geben, um einen elektrischen Strom anzulegen, wenn eine statistisch signifikative Verringerung der Impedanz identifiziert wird.

4. Rückenmarksstimulationssystem nach einem der vorhergehenden Ansprüche, wobei die Elektrode (1) mit einem Durchmesser ≤ 1,3 mm, welche im Wesentlichen aus einer Vielzahl von Polen (2) besteht, dazu ausgebildet ist, innerhalb des intraduralen Raums (3) über die Dura mater (4) mittels Nadelpunktion (5) der Haut eingeführt zu werden.

5. Rückenmarksstimulationssystem nach einem der vorhergehenden Ansprüche, wobei die Elektrode (1) einen Durchmesser von 0,15 mm bis 0,9 mm aufweist.

6. Rückenmarksstimulationssystem nach einem der vorhergehenden Ansprüche, wobei die Elektrode (1) flexibel ist, um sich durch den intraduralen Raum bis zum gewünschten metamerisches Niveau zur Stimulierung einfach zu bewegen und Schäden im Bereich zu vermeiden.

7. Rückenmarksstimulationssystem nach einem der vorhergehenden Ansprüche, wobei die Elektrode (1) mit einer Abdeckung (7) gedeckt ist, welche ein Trauma verhindert, zum Beispiel Silikonkautschuk-Elastomer.

8. Rückenmarksstimulationssystem nach einem der vorhergehenden Ansprüche, wobei der Durchmesser der Nadel (5) ≤ 1,31 mm, vorzugsweise ≤ 0,91 mm ist.

9. Rückenmarksstimulationssystem nach einem der vorhergehenden Ansprüche, wobei jeder Pol programmiert sein kann, um aktiv oder passiv zu sein.

## Revendications

1. Système de stimulation de moelle épinière qui comprend :
a. une électrode (1) avec un diamètre ≤ 1,3 mm, constituée essentiellement d'une pluralité de pôles (2), apte à être insérée à l'intérieur de l'espace intradural (3) ;
b. unité (6), connectée à l'électrode, configurée pour lire systématiquement l'impédance des pôles (2) lorsque l'électrode (1) traverse la dure-mère (4), et pour générer du courant électrique ; et
c. un ordinateur comprenant une unité de traitement (10) configuré pour :
i. recevoir, depuis l'unité (6), des lectures d'impédance continues de chaque pôle lorsque l'électrode (1) traverse la dure-mère (4),
ii. traiter les lectures d'impédance reçues pour trouver une variation ou une déviation statistiquement significative de l'impédance, et
iii. dans lequel l'ordinateur comprenant une unité de traitement (10) est **caractérisé en ce qu'**il est configuré en outre pour fournir une sortie à travers un terminal et donner des instructions à l'unité (6) pour appliquer du courant électrique à l'intérieur de l'espace intradural (3), lorsqu'une réduction statistiquement significative de l'impédance d'un pôle spécifique (2) est identifiée, qui indique que le pôle (2) a traversé la dure-mère (4) et est placé dans l'espace intradural (3) à la partie interne de la dure-mère-arachnoïde (8).

2. Système de stimulation de moelle épinière, selon la revendication 1, comprenant en outre une fixation externe au tissu (9) pour fixer l'électrode (1) une fois que le pôle est placé dans l'espace intradural à la partie interne de la dure-mère-arachnoïde (8), dans lequel le courant électrique doit être appliqué.

3. Système de stimulation de moelle épinière, selon l'une quelconque des revendications précédentes, dans lequel l'unité de traitement (10) comprend au moins un programme informatique comprenant des instructions pour recevoir, depuis l'unité (6), des lectures d'impédance continues de chaque pôle, traiter les lectures d'impédance reçues pour trouver une variation ou une déviation statistiquement significative de l'impédance, et fournir une sortie à travers un terminal, et/ou donner des instructions à l'unité (6) pour appliquer du courant électrique, lorsqu'une réduction statistiquement significative de l'impédance est identifiée.

4. Système de stimulation de moelle épinière, selon l'une quelconque des revendications précédentes, dans lequel l'électrode (1) avec un diamètre ≤ 1,3 mm, constituée essentiellement d'une pluralité de pôles (2), est configurée pour être insérée à l'intérieur de l'espace intradural (3) à travers la dure-mère (4) par piqûre par aiguille (5) de la peau.

5. Système de stimulation de moelle épinière, selon l'une quelconque des revendications précédentes, dans lequel l'électrode (1) a un diamètre allant de 0,15 mm à 0,9 mm.

6. Système de stimulation de moelle épinière, selon l'une quelconque des revendications précédentes, dans lequel l'électrode (1) est flexible afin de se déplacer facilement à travers l'espace intradural jusqu'au niveau métamérique souhaité pour stimulation et éviter des dommages dans la zone.

7. Système de stimulation de moelle épinière, selon l'une quelconque des revendications précédentes, dans lequel l'électrode (1) est recouverte d'une enveloppe (7) qui empêche des traumatismes, par exemple en l'élastomère de caoutchouc de silicone.

8. Système de stimulation de moelle épinière, selon l'une quelconque des revendications précédentes, dans lequel le diamètre de l'aiguille (5) est ≤ 1,31 mm, préférablement ≤ 0,91 mm.

9. Système de stimulation de moelle épinière, selon l'une quelconque des revendications précédentes, dans lequel chaque pôle peut être programmé pour être actif ou passif.
